# EUROPEAN PATENT APPLICATION

(11) **EP 4 729 004 A1**
(43) Date of publication of application: **22.04.2026**
(21) Application number: 24822420.6
(22) Date of filing: 26.04.2024
(51) Int. Cl.: A61B 17/34, A61N 1/05

(54) **TORQUE TRANSMISSION MECHANISM, ASSEMBLY METHOD, AND CONVEYING DEVICE FOR IMPLANTABLE MEDICAL DEVICE**

(30) Priority: 15.06.2023 CN 202310706881
(71) Applicant: MicroPort Soaring CRM (Shanghai) Co., Ltd., Shanghai 201203 (CN)
(72) Inventor: HE, Haoxue, Shanghai 201203 (CN); CHENG, Zhijun, Shanghai 201203 (CN); SUN, Jiangkai, Shanghai 201203 (CN); WU, Nan, Shanghai 201203 (CN); NING, Yunfeng, Shanghai 201203 (CN)
(74) Representative: Lavoix
(86) International application number: PCT/CN2024/090021
(87) International publication number: WO 2024/255465

(57) **Abstract**

The present invention provides a torque transmission mechanism, a method of assembly thereof and a delivery device for an implantable medical device. The torque transmission mechanism includes a torque transmission member, a torque receiver and a limiting member. The torque transmission member is sleeved over an outer circumference of the torque receiver along an axis thereof and configured to receive a torque from an inner sheath and transfer it to the torque receiver. If a torque on the torque transmission member does not exceed a rated value, a torque on the torque receiver is equal to the torque on the torque transmission member, and the torque transmission member drives the torque receiver to rotate in synchronization therewith. If the torque on the torque transmission member exceeds the rated value, the torque transmission member rotates around the torque receiver so that the torque on the torque receiver does not exceed the rated value. The limiting member is configured to limit a position of the torque transmission member relative to the torque receiver on the axis of the torque receiver. With this arrangement, it can be ensured that the torque transmission member can only transfer a torque not exceeding the rated value to the torque receiver.

## Description

### TECHNICAL FIELD

The present invention relates to the technical field of medical devices, and in particular to a torque transmission mechanism, a method of assembly thereof and a delivery device for an implantable medical device.

### BACKGROUND

At present, most leadless pacemakers are implanted and fixed either by helix-based active fixation, or by tine-based fixation. During implantation of a helix-based active fixation leadless pacemaker, in order to prevent perforation of cardiac tissue by an excessively screwed distal helix portion, a torque-limiting structure is usually added to the leadless pacemaker, or to an associated delivery device, which ensures that a torque transferred from the delivery device to the leadless pacemaker is less than the torque necessary for the distal helix portion of the leadless pacemaker to penetrate the cardiac tissue.

A leadless pacemaker can be implanted to regulate heart physiological pacing function by delivering the leadless pacemaker into one ventricle and anchoring it to the ventricular septum using a dedicated delivery device. Main steps of this implantation process are as follows. A surgeon assembles the leadless pacemaker with the torque-limiting structure on site in an operating room and then manipulates a torque catheter within a handle to retract the leadless pacemaker into a casing of the delivery device. The delivery device is then inserted into a guide sheath, with which an access has been established. After the casing that houses the leadless pacemaker is advanced into the right atrium, the sheath is steered to allow smooth passage of the casing through the tricuspid valve into the right ventricle. A contrast agent is then injected to confirm that the casing is distally perpendicular to a middle or lower portion of the ventricular septum. The torque catheter is pushed to advance the docking cap to urge the distal helix portion of the leadless pacemaker out of the casing, and then turned to screw the distal helix portion into the ventricular septum. The torque catheter is further manipulated to detach the torque-limiting structure.

The casing is withdrawn from the ventricle into the atrium before testing is conducted with electrical signals, with only a safety steel wire remaining coupled to the leadless pacemaker. Once it is confirmed that the leadless pacemaker functions properly according to the signal testing results, the safety steel wire is separated from the leadless pacemaker, and the delivery device and guide sheath are withdrawn. If the signal testing results indicate malfunctioning, the casing is again pushed from the atrium into the ventricle, and the above implantation steps are repeated.

The above leadless pacemaker implantation process is prone to failure due to the following risks or drawbacks that it is associated with.

Firstly, it requires the surgeon's on-site assembly of the torque-limiting structure, leading to an extended period of time being required for the entire surgical procedure.

Secondly, if the detachment of the torque-limiting structure fails to occur as designed after the leadless pacemaker is anchored onto the ventricular septum, the following problems may arise:
a. Undesirably manipulated (e.g., retracted for withdrawal) of the torque catheter may cause the torque-limiting structure to directly pull the leadless pacemaker off from the interventricular septum, possibly resulting in severe damage to the ventricular septum.
b. The torque catheter may be undesirably turned to unscrew the distal helix part of the leadless pacemaker off the myocardial tissue. Although this may not cause damage to the ventricular septum, it necessitates retrieval of the leadless pacemaker and the delivery device and replacement for new ones before the implantation can be reattempted, resulting in an extended surgical time and a higher surgical risk.

Thirdly, during the retraction of the detached torque-limiting structure back into the casing, it tends to scratch myocardial tissue including trabeculae, the ventricular septum, the ventricular lateral wall or tricuspid chordae tendineae.

### SUMMARY OF THE INVENTION

It is an object of the present invention to provide a torque transmission mechanism, a method of assembly thereof and a delivery device for an implantable medical device, which overcome one or more of the problems associated with the implantation of an implantable medical device using a conventional delivery device.

To this end, the present invention provides a torque transmission mechanism configured to cause an implantable medical device to rotate so as to implant the implantable medical device at a target site, wherein the torque transmission mechanism comprises a torque transmission member, a torque receiver and a limiting member.

The torque transmission member is sleeved over an outer circumference of the torque receiver along an axis of the torque receiver, wherein the torque transmission member is configured to receive a torque from an inner sheath and to transfer the torque to the torque receiver, wherein when a torque on the torque transmission member does not exceed a rated value, the torque on the torque receiver is equal to the torque on the torque transmission member and the torque transmission member drives the torque receiver to rotate in synchronization therewith, wherein when the torque on the torque transmission member exceeds the rated value, the torque transmission member rotates around the torque receiver so that a torque on the torque receiver does not exceed the rated value, wherein the limiting member is configured to limit a position of the torque transmission member relative to the torque receiver on the axis of the torque receiver.

Optionally, the torque transmission mechanism may further comprise a docking member and a coupling member, the docking member disposed at a distal end of the torque receiver along the axis of the torque receiver, the coupling member disposed at a proximal end of the torque transmission member along an axis of the torque transmission member.

The limiting member may be coupled to each of the docking member and the coupling member, wherein the docking member and/or the coupling member is/are rotatably coupled to the limiting member, and the docking member limits a position of the limiting member on the axis of the torque receiver and the coupling member limits a position of the limiting member on the axis of the torque transmission member.

Optionally, one of the coupling member and the limiting member may comprise a circumferential first limiting groove, with the other comprising a circumferential first protruding portion, wherein the first protruding portion is engaged in the first limiting groove, thereby limiting a relative position of the coupling member and the limiting member on the axis of the torque transmission member.

Alternatively or additionally, one of the docking member and the limiting member may comprise a circumferential second limiting groove, with the other comprising a circumferential second protruding portion, wherein the second protruding portion is engaged in the second limiting groove, thereby limiting a relative position of the docking member and the limiting member on the axis of the torque receiver.

Optionally, the docking member is fixedly coupled to the limiting member by snap engagement or threadedly engagement, wherein the coupling member comprises a circumferential first limiting groove, wherein the limiting member comprises a circumferential first protruding portion that is engaged in the first limiting groove, so that the limiting member is around and rotatably coupled to the coupling member.

Optionally, the limiting member may comprise a barrel section, wherein the barrel section surrounds and shields the torque transmission member along the axis of the torque transmission member.

Optionally, the docking member may comprise a support portion extending radially thereof, wherein a distal end of the barrel section is attached to the support portion, wherein the support portion comprises a first through hole, and/or the barrel section comprises a second through hole, wherein the limiting member further comprises a tapered section at a proximal end of the barrel section, wherein the tapered section is tapered away from the support portion and comprises a third through hole, wherein the first through hole and/or the second through hole is/are brought into communication with the third through hole through a first cavity of the barrel section.

Optionally, the torque transmission mechanism may further comprise a docking member disposed at a distal end of the torque receiver, wherein the docking member comprises a second cavity for docking with the implantable medical device, wherein a distal end of the second cavity is open to allow the implantable medical device to be moved into or out of the docking member along an axis thereof, wherein the second cavity configured to, when a proximal end portion of the implantable medical device is accommodated in the second cavity, limit a circumferential rotation of the implantable medical device.

Optionally, a cross-section of the second cavity comprises a first reference axis and a second reference axis that pass through a center of the cross-section, wherein the first reference axis is perpendicular to the second reference axis, wherein the cross-section is symmetrical about each of the first reference axis and the second reference axis, wherein a dimension of the cross-section along the first reference axis is different from a dimension of the cross-section along the second reference axis.

Optionally, the torque transmission mechanism may further comprise a docking member disposed at a distal end of the torque receiver, the docking member comprising a plurality of notches for docking with the implantable medical device, the notches arranged circumferentially around an axis of the docking member, the notches configured to engage the implantable medical device along the axis of the docking member so as to limit a circumferential rotation of the implantable medical device.

Optionally, there may be an interference fit formed between the torque transmission member and the torque receiver, wherein when the torque on the torque transmission member does not exceed the rated value, the torque transmission member and the torque receiver are stationary relative to each other, wherein when the torque on the torque transmission member exceeds the rated value, the torque transmission member overcomes friction between it and the torque receiver and hence rotates around the torque receiver.

Optionally, the torque transmission member may comprise a torque spring, wherein the torque receiver comprises a cylindrical outer circumferential wall, and wherein the torque spring is wound on the outer circumferential wall.

Optionally, the torque transmission member comprises a fourth through hole axially extending therethrough, wherein the torque receiver comprises a fifth through hole axially extending therethrough, wherein the fourth and fifth through holes are configured for passage of a safety wire therethrough.

To the above end, the present invention also provides a method of assembling the torque transmission mechanism as defined above. The method comprises:
disposing the torque transmission member over the torque receiver;
performing a torque test on the torque transmission member and the torque receiver by applying a torque exceeding the rated value to the torque transmission member, wherein if the torque transmission member rotates around the torque receiver, the torque test is passed; and
after passing the torque test, coupling the limiting member to each of the torque transmission member and the torque receiver so that a position of the torque transmission member is limited relative to the torque receiver on the axis of the torque receiver.

Optionally, the method may further comprise:
wherein before the torque transmission member is disposed over the torque receiver, the coupling member is assembled with and coupled to a proximal end of the torque transmission member; and
wherein after the torque test is passed, the docking member at a distal end of the torque receiver is fixedly coupled to the limiting member and the first protruding portion of the limiting member is engaged into the first limiting groove of the coupling member so that the limiting member is rotatably coupled to the coupling member.

To the above end, the present invention also provides a delivery device for an implantable medical device, comprising the torque transmission mechanism as defined above, an outer sheath, an inner sheath and a safety wire,
wherein the inner sheath is movably disposed inside the outer sheath, wherein the safety wire is movably disposed inside each of the inner sheath and the torque transmission mechanism, wherein the inner sheath is coupled to the torque transmission member of the torque transmission mechanism and is configured to actuate the torque receiver of the torque transmission mechanism by transferring a torque to the torque transmission member,
wherein a distal end of the torque transmission mechanism is configured to be detachably coupled to the implantable medical device, and wherein the torque receiver of the torque transmission mechanism is coupled to the implantable medical device so as to limit a circumferential rotation of the implantable medical device,
wherein the safety wire is configured to be detachably coupled to the implantable medical device.

In summary, the present invention provides a torque transmission mechanism, a method of assembly thereof and a delivery device for an implantable medical device. The torque transmission mechanism includes a torque transmission member, a torque receiver and a limiting member. The torque transmission member is sleeved over an outer circumference of the torque receiver along an axis thereof and is configured to receive and transfer a torque from an inner sheath to the torque receiver. If a torque on the torque transmission member does not exceed a rated value, a torque on the torque receiver is equal to the torque on the torque transmission member, and the torque transmission member drives the torque receiver to rotate in synchronization therewith. Otherwise, if the torque on the torque transmission member exceeds the rated value, the torque transmission member rotates around the torque receiver so that the torque on the torque receiver does not exceed the rated value. The limiting member is configured to limit a position of the torque transmission member relative to the torque receiver on the axis of the torque receiver.

With this arrangement, according to the configuration of the torque transmission member and the torque receiver, the torque transmission member, when a torque thereon exceeds the rated value, will rotationally slip on and around the torque receiver, thereby ensuring that the torque transmission member can only transfer a torque not exceeding the rated value to the torque receiver. Additionally, it is unnecessary to separate the torque transmission member from the torque receiver, preventing possible introduction of a gap which might occur if the separation fails. The provision of the limiting member ensures that the torque transmission member will not axially separate from the torque receiver, thus ensuring reliability of the entire torque transmission mechanism and eliminating the risk of scratching of myocardial tissue that might be caused by separation of the torque transmission member from the torque receiver.

### BRIEF DESCRIPTION OF THE DRAWINGS

Those of ordinary skill in the art will understand that the accompanying drawings are presented to enable a better understanding of the present invention and not intended to limit the scope thereof in any sense, in which:
Fig. 1 schematically illustrates a delivery device according to an embodiment of the present invention during its use with an implantable medical device, in which the implantable medical device is separated from a docking member, while a safety wire is coupled to the implantable medical device.
Fig. 2 schematically illustrates a delivery device according to an embodiment of the present invention during its use with an implantable medical device, in which the implantable medical device is coupled to a docking member, with a safety wire being coupled to the implantable medical device.
Fig. 3 shows a schematic axial cross-sectional view of an implantable medical device according to an embodiment of the present invention being housed in a casing.
Fig. 4 shows a schematic diagram of a torque transmission mechanism according to an embodiment of the present invention.
Fig. 5 shows a schematic axial cross-sectional view of a torque transmission mechanism according to an embodiment of the present invention.
Figs. 6a to 6c show schematic distal end views of three preferred examples of a torque transmission mechanism according to an embodiment of the present invention.
Fig. 7 shows a schematic axial cross-sectional view of another preferred example of the torque transmission mechanism, in which coupling of a docking member and a limiting member is accomplished by snap engagement.
Fig. 8 shows a schematic axial cross-sectional view of yet another preferred example of the torque transmission mechanism, in which coupling of a docking member and a limiting member is accomplished by a threaded connection.
Fig. 9 shows a schematic axial cross-sectional view of still yet another preferred example of the torque transmission mechanism.
Fig. 10 shows a schematic diagram of a further preferred example of the torque transmission mechanism.

### Reference Numerals

1 implantable medical device; 11 distal portion; 12 proximal portion; 2 torque transmission mechanism; 21 torque transmission member; 211 fourth through hole; 22 torque receiver; 221 fifth through hole; 23 limiting member; 230 first cavity; 231 barrel section; 232 tapered section; 233 third through hole; 24 docking member; 240 second cavity; 241 support portion; 242 first through hole; 243 guide chamfer; 244 notch; 25 coupling member; 250 body; 251 stop shoulder; 252 limiting block; 253 sixth through hole; 3 outer sheath; 4 inner sheath; 5 safety wire; 6 casing; 71 first limiting groove; 72 first protruding portion.

### DETAILED DESCRIPTION

Objects, advantages and features of the present invention will become more apparent upon reading the following more detailed description of specific embodiments thereof in conjunction with the accompanying drawings. Note that the figures are provided in a very simplified form not necessarily drawn to exact scale for the only purpose of helping explain embodiments of the invention disclosed herein in a more convenient and clearer way. In addition, the illustrated structures are usually part of their real-world counterparts. In particular, as the figures tend to have distinct emphases, they are sometimes drawn to different scales.

As used herein, the singular forms "a", "an" and "the" include plural referents, and the term "or" is generally employed in the sense of "and/or", "a number of" is generally employed in the sense of "at least one" and "at least two" is generally employed in the sense of "two or more". Additionally, the use of the terms "first", "second" and "third" herein is intended for illustration only and is not to be construed as denoting or implying relative importance, or as implicitly indicating the number of referenced items. Accordingly, defining an item with "first", "second" or "third" is an explicit or implicit indication of the presence of one or at least two such items. The terms "one end" and "other end", as well as "proximal end" and "distal end", may be used herein to generally refer to portions around two opposite ends, rather than only to the ends. As used herein, the terms "proximal end" and "distal end" may be defined with respect to a delivery device for an implantable medical device, one end of which is inserted inside a human body, with the other end remaining outside the body for manipulation. In this regard, the term "proximal end" refers to a location closer to the manipulation end of the delivery device outside the human body, while the term "distal end" refers to a location, which is closer to the end of the delivery device that is inserted within the human body and therefore farther away from the manipulation end of the delivery device. Alternatively, in a manual operation or an operation conducted by hand, the terms "proximal end" and "distal end" may also be defined with respect to an operator, such as a surgeon or clinician. In this regard, the term "proximal end" refers to a location closer to an operator, while the term "distal end" refers to a location, which is closer to the delivery device for the implantable medical device and therefore farther away from the operator. Further, as used herein, the terms "mounting", "coupling", "connecting", "disposing" and any variants thereof should be interpreted in a broad sense. When an element is referred to as being "disposed" on another element, this is generally intended to mean that there is a connection, coupling, engagement or transmission relationship between the two elements, which may be either direct or indirect with one or more intervening elements, and should not be interpreted as indicating or implying a particular spatial position relationship between them. That is, the element may be located inside, outside, above, under, beside, or at any other location relative to the other element, unless the context clearly dictates otherwise. Those of ordinary skill in the art can understand the specific meanings of the above-mentioned terms herein, depending on their context. Furthermore, the directional terms such as "above", "below", "upper", "lower", "upward", "downward", "left", "right", and the like are used in relation to the illustrative embodiments as they are depicted in the figures, the upward or upper direction being toward the top of the corresponding figure and the downward or lower direction being toward the bottom of the corresponding figure.

As described below with reference to the accompanying drawings, the present invention seeks to provide a torque transmission mechanism, a method of assembly thereof and a delivery device for an implantable medical device, which overcome one or more of the problems associated with the implantation of an implantable medical device using a conventional delivery device.

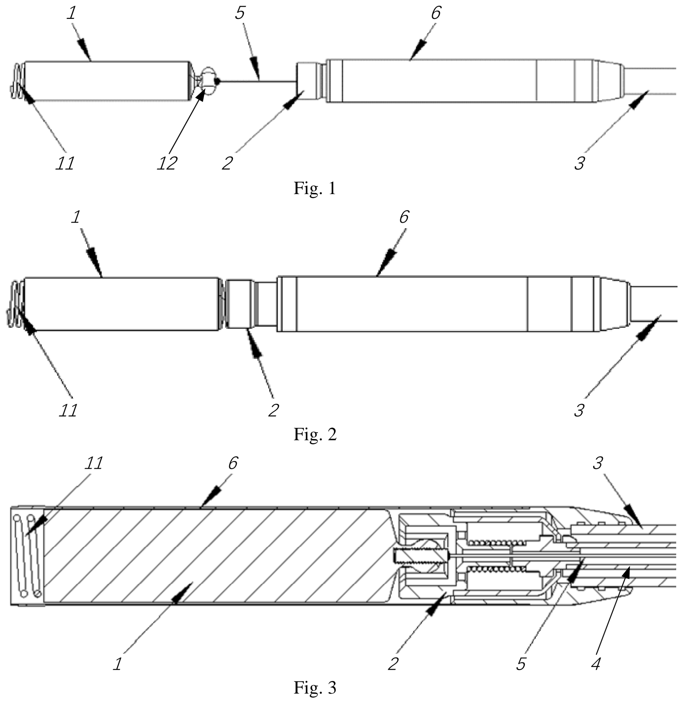

Referring to Figs. 1 to 3, in embodiments described herein, there is provided a delivery device for an implantable medical device. It is used to deliver the implantable medical device 1 (e.g., a leadless pacemaker) and implant it at a target site (e.g., the ventricular septum in the heart). The delivery device includes a torque transmission mechanism 2, an outer sheath 3, an inner sheath 4 and a safety wire 5. The inner sheath 4 is movably disposed inside the outer sheath 3, with the safety wire 5 being movably disposed inside both the inner sheath 4 and the torque transmission mechanism 2. The inner sheath 4 is coupled to a torque transmission member 21 of the torque transmission mechanism 2 and configured to actuate a torque receiver 22 of the torque transmission mechanism 2. The torque transmission mechanism 2 is configured to be detachably coupled at a distal end thereof to the implantable medical device 1. Coupling the torque receiver 22 of the torque transmission mechanism 2 to the implantable medical device 1 restricts circumferential rotation of the implantable medical device 1. The safety wire 5 is configured for separable coupling to the implantable medical device 1, which locks the implantable medical device 1 and prevents it from falling off during delivery. The safety wire 5 may be separated from the implantable medical device 1 after the implantable medical device 1 is implanted and passes an associated test.

The structure and operation of the delivery device for the implantable medical device 1 will be described with reference to Figs. 1 to 3. As shown in Figs. 1 and 2, in one exemplary example, a distal portion 11 of the implantable medical device 1 comprises the form of a helix for screwing at the target site, and results in fixation of the entire implantable medical device 1 at the target site. A proximal portion 12 of the implantable medical device 1 is detachably coupled to the safety wire 5, for example, by a threaded connection. It will be appreciated that the implantable medical device 1 can be separated from the safety wire 5, when so desired, by turning the safety wire 5 so as to separate the safety wire 5 from the proximal portion 12 of the implantable medical device 1.

When assembled with and coupled to the proximal portion 12 of the implantable medical device 1, the safety wire 5 moves forward and backward along its own axis (oriented horizontally as viewed in the orientation of Fig. 1) to cause the implantable medical device 1 move forward and backward along the axis. Fig. 1 shows the implantable medical device 1 being separated from a docking member 24 of the torque transmission mechanism 2. From this configuration of Fig. 1, proximally pulling the safety wire 5 (to the right as viewed in the orientation of Fig. 1) can cause the implantable medical device 1 to approach the torque transmission mechanism 2 and ultimately couple the implantable medical device 1 to the torque transmission mechanism 2. Once this occurs, the implantable medical device 1 is restrained from circumferential rotation relative to the torque transmission mechanism 2, as shown in Fig. 2.

As noted above, the distal portion 11 of the implantable medical device 1 is configured for screwing at the target site and hence fixation of the entire implantable medical device 1 there. However, the safety wire 5 is usually thin and therefore not able to transfer a significant torque. Therefore, the torque transmission mechanism 2 is provided to transfer the torque, allowing the implantable medical device 1 to rotate as a whole about its own axis and hence accomplish the screwing of the distal portion 11 at the target site. Once the safety wire 5 is pulled proximally (to the right as viewed in the orientation of Fig. 1), the implantable medical device 1 forms a connection with the torque transmission mechanism 2, so that a torque transfer is made possible from the torque transmission mechanism 2 to the implantable medical device 1.

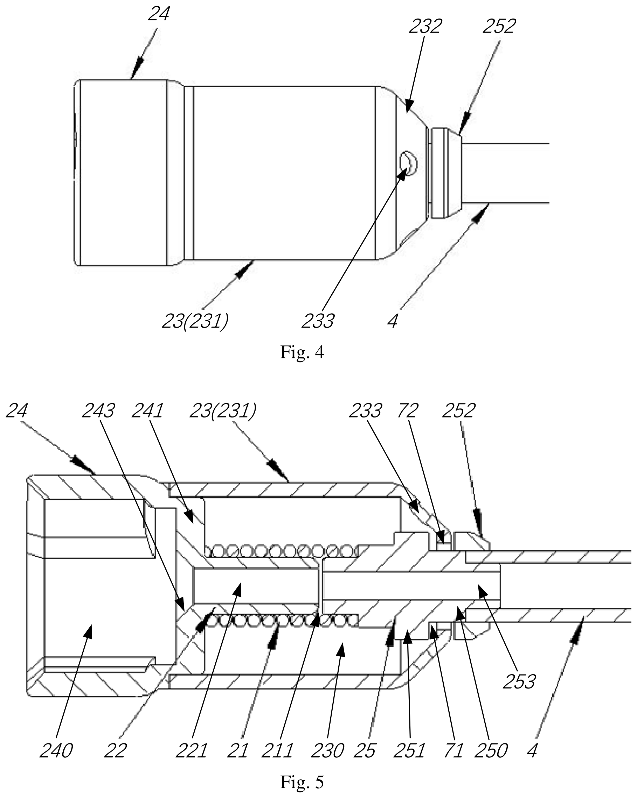

Further, referring to Fig. 3, the inner sheath 4 may be, for example, a tube with some degree of twist resistance, such as a braided tube, a multi-strand coil spring, or a double-layer tube. Once the torque transmission member 21 of the torque transmission mechanism 2 is coupled to the inner sheath 4 (e.g., as shown in Fig. 5), if the inner sheath 4 exerts a torque not exceeding a rated value on the torque transmission mechanism 2, the torque from the inner sheath 4 can be transferred via the torque transmission mechanism 2 to the torque receiver 22 and hence to the implantable medical device 1. Thus, rotation of the inner sheath 4 about its own axis causes the implantable medical device 1 to rotate about the axis.

Optionally, as shown in Fig. 3, the delivery device for the implantable medical device 1 further includes a casing 6, which is coupled at a proximal end thereof (e.g., its right hand end as viewed in the orientation of Fig. 3) to the outer sheath 3 and comprises a distally open cavity for accommodating the implantable medical device 1 and the torque transmission mechanism 2. Referring to Figs. 2 and 3, the inner sheath 4 is also movable relative to the outer sheath 3 along an axis thereof, causing the torque transmission mechanism 2 to also move along the axis of the outer sheath 3. This, working with the safety wire 5, enables the implantable medical device 1 and the torque transmission mechanism 2 to be together received in the casing 6, forming a configuration before implantation. Preferably, the outer sheath 3 is a steerable sheath, and placement of the casing 6 into a guide sheath may follow for delivery and implantation.

Referring to Figs. 4 and 5, embodiments described herein provide a torque transmission mechanism 2, which overcomes the problems associated with the implantation of conventional delivery devices. According to specific embodiments, the torque transmission mechanism 2 is used to rotate an implantable medical device 1 so that its distal portion, which is, for example, a distal portion of a helix, is anchored at a target site. The torque transmission mechanism 2 includes a torque transmission member 21, a torque receiver 22 and a limiting member 23. The torque transmission member 21 is sleeved onto an outer circumference of the torque receiver 22 along an axis thereof and configured to receive a torque from an inner sheath 4 and transmit the torque to the torque receiver 22. If a torque on the torque transmission member 21 does not exceed a rated value, a torque on the torque receiver 22 is the same as the torque on the torque transmission member 21, and the torque transmission member 21 drives the torque receiver 22 to rotate in sync therewith. If a torque on the torque transmission member 21 exceeds the rated value, the torque transmission member 21 rotates around the torque receiver 22 so that a torque on the torque receiver 22 does not exceed the rated value. The limiting member 23 is configured to limit the position of the torque transmission member 21 relative to the torque receiver 22 along the axis thereof.

As mentioned herein, the rated value refers to a value at which the maximum rotational frictional torque between the torque transmission member 21 and the torque receiver 22 reaches equilibrium. It may also be referred to as a maximum torque rating, and also represents the torque required for myocardial implantation.

In one application of the delivery device, the torque transmission member 21 is located at a proximal end for receiving an input torque and, for example, coupled to the inner sheath 4, while the torque receiver 22 is located at a distal end for outputting torque and, for example, coupled to the implantable medical device 1.

It should be noted that the torque on the torque transmission member 21 is a rotating torque caused on the torque transmission member 21 by an external drive torque, for example, from the inner sheath 4. When the inner sheath 4 is rotated, a torque will be exerted on the torque transmission member 21, creating a tendency of the transmission member 21 to rotate. If a resistive torque from the outside or from an external structure on the torque receiver 22 is less than a maximum torque rating for the torque transmission member 21, the torque transmission member 21 will rotate about its own axis, causing the torque receiver 22 to simultaneously rotate in the same direction, so that the two remain stationary relative to each other. If a resistive torque from the outside or from an external structure on the torque receiver 22 is not less than the maximum torque rating for the torque transmission member 21, then the torque transmission member 21 will not cause synchronous rotation of the torque receiver 22, the torque transmission member 21 will rotate under the action of a torque from the inner sheath 4 but the torque receiver 22 remains stationary, and the torque transmission member 21 slips on a circumference of the torque receiver 22. Therefore, the two move circumferentially relative to each other. The resistive torque from the outside mainly results from the reactive torque generated when the implantable medical device 1 is screwed into the myocardial tissue.

In another application of the delivery device, for example, following successful screwing of the distal portion 11 of the implantable medical device 1 at a target site, the inner sheath 4 outputs an increasing torque. When the torque from the torque transmission member 21 increases above the rated value, the equilibrium of the rotational friction torque between the torque transmission member 21 and the torque receiver 22 will be overcome, and the torque transmission member 21 starts to rotationally slip relative to the torque receiver 22. Therefore, a torque on the torque receiver 22 will not exceed the rated value, preventing the distal portion 11 of the implantable medical device 1 from applying an excessive torque to the target site, which may cause perforation of cardiac tissue there. The rated value may be selected as any practically desired value by those skilled in the art. According to our findings obtained from extensive studies and repeated experimental demonstrations, a torque of approximately 0.2 N·cm is generally sufficient for the implantation of the implantable medical device 1, and perforation of cardiac tissue may occur at a torque exceeding 1 N-cm. Accordingly, the rated value may be selected as a value, which is greater than 0.2 N·cm and less than the perforating torque while providing a safety margin. Therefore, it may be selected as a value above 0.2 N·cm, such as 0.5 N·cm or 0.6 N·cm. Of course, those skilled in the art may select the rated value as different value, depending on the age and physical condition of a patient.

As discussed above, the torque transmission member 21 and the torque receiver 22 are configured so that the torque transmission member 21 will, when a torque exerted thereon exceeds the rated value, rotationally slip over the torque receiver 22, guaranteeing a torque below the rated value transferred from the torque transmission member 21 to the torque receiver 22. Additionally, it is unnecessary to separate the torque transmission member 21 from the torque receiver 22, preventing possible introduction of a gap which might occur if the separation fails. The provision of the limiting member 23 ensures that the torque transmission member 21 will not separate from the torque receiver 22 along the axis of the torque receiver 22, thus ensuring reliability of the entire torque transmission mechanism 2 and eliminating the risk of scratching of myocardial tissue that might be caused by separating torque transmission member 21 from the torque receiver 22.

Preferably, there is an interference fit between the torque transmission member 21 and the torque receiver 22 such that the torque transmission member 21 and the torque receiver 22 remain stationary relative to each other when a torque on the torque transmission member 21 does not exceed the rated value and that the torque transmission member 21 overcomes friction between it and the torque receiver 22 and therefore rotates over the torque receiver 22 when a torque on the torque transmission member 21 exceeds the rated value. It should be noted that although the torque transmission member 21 and the torque receiver 22 have been described above as being coupled by an interference fit, this is merely a preferred example and the coupling is not limited to being accomplished by any particular approach. In some embodiments, the coupling between the torque transmission member 21 and the torque receiver 22 may be accomplished by self-restoring coupling methods with torque transfer capabilities, such as those based on magnetic coupling, friction-based docking or slip teeth. All these coupling methods capable of torque transfer are also able to provide to a limited torque output in response to torque overload, and the present invention is not limited in this regard.

In the present embodiment, the torque receiver 22 comprises a cylindrical outer circumferential wall, and the torque transmission member 21 includes a torque spring wound on the outer circumferential wall of the torque receiver 22. In an initial state of the torque spring without any force being applied thereto, its inner diameter is slightly less than an outer diameter of the outer circumferential wall of the torque receiver 22, thus allowing the torque spring wound on the outer circumferential wall to form an interference fit. Optionally, the torque spring may be made of a single wire wound with a specified pitch, direction and length. The wire may be 304 or 316 stainless steel. Due to the interference fit between the outer circumferential wall of the torque receiver 22 and the torque spring wound thereon, there is friction between the two, which allows a torque not exceeding the rated value on the torque spring to be transferred to the torque receiver 22 at a ratio of 1: 1. However, when a torque on the torque spring exceeds the rated value, the torque spring overcomes the friction, and circumferentially slip over and relative to the outer circumferential wall. Since the torque receiver 22 is also involved in torque transfer, it is desired to exhibit some degree of torsion resistance and is therefore preferably made of a metal material, such as 304 or 316 stainless steel.

With continued reference to Figs. 4 and 5, in an alternative exemplary example, the torque transmission mechanism 2 further includes a docking member 24 and a coupling member 25. The docking member 24 is disposed at a distal end of the torque receiver 22 along its axis, and the coupling member 25 is disposed at a proximal end of the torque transmission member 21 along its axis. The limiting member 23 is coupled to both the docking member 24 and the coupling member 25. The docking member 24 and/or the coupling member 25 may be rotatably coupled to the limiting member 23, with the docking member 24 limiting the position of the limiting member 23 on the axis of the torque receiver 22 and the coupling member 25 limiting the position of the limiting member 23 on the axis of the torque transmission member 21. With combined reference to Figs. 1 to 3, the docking member 24 is configured essentially to dock with, and transfer a torque to, the proximal portion 12 of the implantable medical device 1. The coupling member 25 is configured for coupling to the inner sheath 4 and for transfer of a torque from the inner sheath 4 to the torque transmission member 21. Of course, in some other embodiments, the docking member 24 may be integrally formed with the torque receiver 22.

At least one of the docking member 24 and the coupling member 25 is rotatably coupled to the limiting member 23 so that the limiting member 23 does not hinder relative rotation of the torque transfer member 21 and the torque receiver 22. The docking member 24 and the coupling member 25 ensures, by means of the limiting member 23, that the torque transfer member 21 and the torque receiver 22 are maintained at fixed relative positions on the axis of the torque receiver 22. Even when relative rotation occurs between the torque transfer member 21 and the torque receiver 22, they will not disengage or separate from each other, eliminating the risk of myocardial tissue being scratched due to separation of the torque transfer member 21 and the torque receiver 22. It should be noted that, in some embodiments, only one of the docking member 24 and the coupling member 25 may be rotatably coupled to the limiting member 23, with the other being fixedly coupled to the limiting member 23. In some other embodiments, both the docking member 24 and the coupling member 25 may be rotatably coupled to the limiting member 23. In every case, decoupled rotation can be achieved.

Optionally, one of the coupling member 25 and the limiting member 23 may comprise a circumferential first limiting groove 71, while the other may comprise a circumferential first protruding portion 72, which is engaged in the first limiting groove 71, thereby limiting relative positions of the coupling member 25 and the limiting member 23 on the axis of the torque transmission member 21. Additionally or alternatively, one of the docking member 24 and the limiting member 23 may comprise a circumferential second limiting groove (not shown), while the other may comprise a circumferential second protruding portion (not shown), which is engaged in the second limiting groove, thereby limiting relative positions of the docking member 24 and the limiting member 23 on the axis of the torque receiver 22.

In the present embodiment, as shown in Fig. 5, the docking member 24 is fixedly coupled to the limiting member 23, and the coupling member 25 is rotatably coupled to the limiting member 23. Specifically, the coupling member 25 comprises a circumferential first limiting groove 71, and the limiting member 23 comprises a circumferential first protruding portion 72, which is engaged in the first limiting groove 71. As a result, the limiting member 23 is around and rotatably coupled to the coupling member 25. For convenience of assembly, the first limiting groove 71 may be formed as a result of sequential assembly of a number of components of the coupling member 25. For example, the coupling member 25 may include a body 250, a stop shoulder 251 radially raised from the body 250 and a separable limiting block 252. The first protruding portion 72 of the limiting member 23 may be, for example, in the form of an annular or a number of circumferentially arranged teeth. The first protruding portion 72 may be rotatably sleeved over the body 250 from the proximal end to the distal end (to the left as viewed in the orientation of Fig. 5), and the limiting block 252 may be then mounted to the body 250 from the proximal end to the distal end until it is located in the vicinity of, or abut against, the first protruding portion 72. Moreover, the limiting block 252 may be then fixed to the body 250 and the inner sheath 4, for example, by welding, melting or gluing. In this way, the first limiting groove 71 can be formed between the stop shoulder 251 and the limiting block 252, with the first protruding portion 72 being engaged in the first limiting groove 71. Preferably, the first protruding portion 72 is annular and has an inner diameter exceeding an outer diameter of the body 250. That is, the coupling member 25 has such a diameter at the first limiting groove 71 that a clearance fit is formed between the first protruding portion 72 and the body 250, which can avoid resistance to rotation. It will be appreciated that the second limiting groove and the second protruding portion may be structured and engaged similar to the first limiting groove 71 and the first protruding portion 72 and, therefore, need not be described in further detail herein.

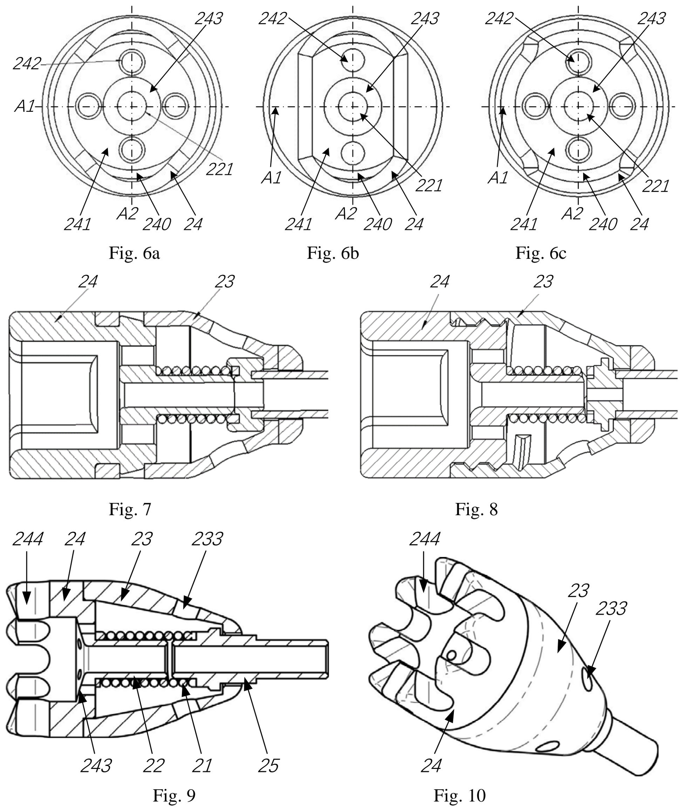

As shown in Fig. 7, in another preferred example, in addition to the rotatable coupling of the coupling member 25 to the limiting member 23, the docking member 24 is fixedly coupled to the limiting member 23 by snap engagement. As such snap engagement requires some degree of elasticity, the limiting member 23 is preferably made of a polymer material, such as polyurethane (PU), polyether block polyamide (PEBAX), polyethylene (PE) or another material satisfies the biocompatible requirements.

As shown in Fig. 8, in another preferred example, in addition to the rotatable coupling of the coupling member 25 to the limiting member 23, the docking member 24 is fixedly coupled to the limiting member 23 by a threaded connection. The docking member 24 may be made of the same metal material as the limiting member 23, such as 304 or 316 stainless steel or a titanium alloy. The docking member 24 and the limiting member 23 would essentially exhibit the same fatigue and other properties, they can be disassembled conveniently and replaced easily. It will be appreciated that both the coupling arrangements of Figs. 7 and 8 allow relative positions of the docking member 24 and the limiting member 23 to be limited on the axis of the torque receiver 22 in the assembled configuration. Of course, the coupling arrangements of Figs. 7 and 8 are also applicable to the coupling of the coupling member 25 and the limiting member 23, and the present invention is not limited in this regard.

Of course, the foregoing embodiments are merely non-limiting examples of the first limiting groove 71 and the first protruding portion 72. In other non-limiting embodiments, the first limiting groove 71 may also be an annular groove integrally formed in the coupling member 25, and the engagement of the first protruding portion 72 of the limiting member 23 in the first limiting groove 71 may also be accomplished by elastic deformation, thermal expansion and contraction, shape memory, integral formation or the like, and the present invention is not limited in this regard. Optionally, once the coupling member 25 is made rotatable relative to the limiting member 23, the docking member 24 may be fixedly coupled to the limiting member 23, for example, by welding, melting or gluing, so as to be rotatable in sync therewith, resulting in easier assembly and more structural simplicity.

In some other embodiments, the rotatable coupling of the docking member 24 and the limiting member 23 may also be accomplished by a second limiting groove and a corresponding second protruding portion provided in and on the limiting member 23 and the docking member 24, respectively. In these embodiments, the coupling member 25 may be fixedly coupled to the limiting member 23 so as to be rotatable in sync therewith. Of course, in some further embodiments, a first limiting groove 71 and a corresponding first protruding portion 72 may be provided in and on the limiting member 23 and the coupling member 25, with a second limiting groove and a corresponding second protruding portion being provided in and on the limiting member 23 and the docking member 24, respectively.

Optionally, as shown in Fig. 5, the limiting member 23 may include a barrel section 231 surrounding the torque transmission member 21 and shielding the torque transmission member 21 along its axial direction. The barrel section 231 may be, for example, cylindrical in shape and have a length measured along the axis of the torque transmission member 21, which is greater than a length of the torque transmission member 21 and the barrel section 231 shields the entire torque transmission member 21 along its axial direction (i.e., the distal and proximal ends of the barrel section 231 may protrude beyond the distal and proximal ends of the torque transmission member 21). As part of the limiting member 23, the barrel section 231 serves to limit relative positions of the docking member 24 and the coupling member 25. In addition, it provides a protective sleeve, which shields the torque transmission member 21 across its axial direction and thereby prevents direct contact between the human tissue and the torque transmission member 21. In the present embodiment, the torque transmission member 21 includes a torque spring with gaps between its adjacent turns. Without such shielding of the torque spring, it would be possible for human tissue to occasionally and hazardously enter gaps between adjacent turns of the torque spring. This can be circumvented by shielding the torque transmission member 21 through the barrel section 231.

Referring to Figs. 5 and 6a to 6c, the docking member 24 includes a support portion 241 extending radially with respect to the docking member 24, and the barrel section 231 is attached at a distal end thereof (i.e., its left hand end as viewed in the orientation of Fig. 5) to the support portion 241. The support portion 241 comprises a first through hole 242, and/or the barrel section 231 comprises a second through hole (not shown). Referring to Figs. 4 and 5, the limiting member 23 further includes a tapered section 232 at a proximal end of the barrel section 231. The tapered section 232 is tapered away from the support portion 241 (to the right as viewed in the orientation of Fig. 5). The tapered section 232 comprises a third through hole 233. The first through hole 242 and/or the second through hole is/are brought into communication with the third through hole 233 through a first cavity 230 of the barrel section 231.

In the present exemplary example, the support portion 241 is a circular plate extending radially with respect to the docking member 24, and the torque receiver 22 is normally arranged at the center of the support portion 241. The tapered section 232 has a conical shape and is tapered away from the support portion 241 and joined to the first protruding portion 72 at a location corresponding to the first limiting groove 71. The tapered section 232 that is tapered away from the support portion 241 can facilitate reducing resistance encountered by the torque transmission mechanism 2 during its movement in blood, which acts parallel to the axis thereof. The barrel section 231 has an inner diameter matching an outer diameter of the support portion 241, and the barrel section 231 can be sleeved over an outer circumference of the support portion 241 and then fixed thereto by welding, melting or gluing. The barrel section 231 comprises a first cavity 230, in which both the torque receiver 22 and the torque transmission member 21 are received. Outer diameters of the torque receiver 22 and the torque transmission member 21 are less than the inner diameter of the barrel section 231, leaving a clearance between the torque transmission member 21 and the barrel section 231.

Further, as shown in Figs. 6a to 6c, the first through hole 242 may, for example, extend through the support portion 241 in a direction parallel to an axis of the support portion 241. The second through hole may, for example, extend through a side wall of the barrel section 231 along a radial direction of the barrel section 231. The third through hole 233 may, for example, extend through a tapered side wall of the tapered section 232. It should be noted that either or both of the first through hole 242 in the support portion 241 and the second through hole in the barrel section 231 may be provided.

With this arrangement, the first through hole 242 and/or the second through hole may be brought into communication with the third through hole 233 through the first cavity 230. In practical applications, the passage(s) from the first through hole 242 and/or the second through hole to the third through hole 233 may be used to facilitate blood circulation, reducing or preventing the formation of blood clots or thrombi within the first cavity 230 and hence the entry of such blood clots or thrombi into the heart through the first through hole 242, the second through hole, the third through hole 233 or other accesses. The torque transmission mechanism 2 often needs to be advanced and retracted within a tubular body having an inner diameter comparable to the size of the torque transmission mechanism 2 (e.g., a blood vessel, a guide sheath, the casing 6, etc.) Consequently, the torque transmission mechanism 2 may encounter, at both axial ends, resistance from liquids (e.g., blood, a contrast agent, physiological saline, etc.) during forward and backward movement of the torque transmission mechanism 2. The provision of the passage(s) from the first through hole 242 and/or the second through hole to the third through hole 233 allows circulation of such liquids in the tubular body, circumventing the resistance generated from the forward and backward movement of the torque transmission mechanism 2. Optionally, two or more first through holes 242 may be preferably provided, such as two, four, six or eight, which may have a diameter selected from the range of 0.6 mm to 1.5 mm, preferably 0.8 mm, 1.0 mm or 1.2 mm, for example. Additionally, two or more third through holes 233 may be preferably provided, such as two, four, six or eight, which may have a diameter preferably not exceeding 2 mm. More preferably, the first through holes 242, the second through holes and the third through holes 233 may be equidistantly and circumferentially arranged, ensuring a relatively consistent blood flow rate within the first cavity 230.

In other embodiments, the first through holes 242 and second through holes may be omitted, and only the third through holes 233 may be provided. Alternatively, the first through holes 242 and the third through holes 233 may be omitted, and only the second through holes may be provided. Further description of such embodiments is omitted herein.

With continued reference to Fig. 5, in conjunction with Figs. 6a to 6c, the docking member 24 may optionally comprise a second cavity 240 for docking with the implantable medical device 1. The second cavity 240 has an open distal end (i.e., the left hand end thereof as viewed in the orientation of Fig. 5), which allows the implantable medical device 1 to be moved into or out of the docking member 24 along an axis thereof. The second cavity 240 may be configured to, when a proximal end portion of the implantable medical device 1 (e.g., the proximal portion 12 of the implantable medical device 1 as shown in Fig. 1) is accommodated in the second cavity 240, limit circumferential rotation of the implantable medical device 1. Since it is not necessary for the torque transmission mechanism 2 to limit a transferred torque by its own separation according to the present embodiment, it is desired to be easily attached or detached to or from the implantable medical device 1 to facilitate implantation or retrieval of the implantable medical device 1. In this sense, through configuring the distal end of the second cavity 240 to be open, it will no longer limit movement of the implantable medical device 1 along its own axis, and limiting the position of the implantable medical device 1 on its own axis will rely simply on the safety wire 5. Accordingly, when not restrained by the safety wire 5, the implantable medical device 1 is able to freely move into or out of the second cavity 240 along the axis of the docking member 24. As such, once the implantable medical device 1 is screwed into the ventricular septum, even if the inner sheath 4 is errorously manipulated (e.g., retracted), as the implantable medical device 1 is coupled only to the safety wire 5 and is separate from the torque transmission mechanism 2, the inner sheath 4 will only cause proximal movement of the torque transmission mechanism 2, instead of pulling the implantable medical device 1 off the ventricular septum, eliminating the risk of damage to the ventricular septum. Further, the torque transmission mechanism 2 that is separate from the implantable medical device 1 can be easily recoupled to the implantable medical device 1 simply by sleeving the second cavity 240 over the proximal portion 12 of the implantable medical device 1 along the axis of the docking member 24. In this way, even if an initial implantation attempt fails, the implantable medical device 1 can be easily retrieved for another implantation.

Optionally, as shown in Fig. 5, the torque transmission member 21 may comprise a fourth through hole 211 extending through itself along its own axis, and the torque receiver 22 may comprise a fifth through hole 221 extending through itself along its own axis. In one embodiment, the torque transmission member 21 may include a torque spring, wherein the fourth through hole 211 is an inner bore forming as a result of coiling the torque spring. The torque receiver 22 may be a circular tubular member, wherein the fifth through hole 221 may be provided by an inner bore of the tubular member. The coupling member 25 may comprise a sixth through hole 253 extending through itself along its own axis. The sixth through hole 253, the fourth through hole 211 and the fifth through hole 221 may be coaxial with one another, and the safety wire 5 may be passed through them.

The fifth through hole 221 may have a diameter selected from the range of 0.6 mm to 1.2 mm, preferably 0.6 mm, 0.8 mm or 1.0 mm. Additionally, the docking member 24 comprises a flared guide chamfer 243 at a distal end of the fifth through hole 221. The size of the flared structure increases away from the coupling member 25 to facilitate retraction of the safety wire 5.

In another embodiment, the torque transmission member 21 may also be in the form of a barrel-like rubber tube nested with and fixed to a circular hard sleeve. The hard sleeve is disposed over the barrel-like rubber tube. The torque receiver 22 may be in the form of a circular tubular member, and a distal portion of the barrel-like rubber tube may be sleeved over the torque receiver 22. In this case, the fourth through hole 211 may be provided by an inner bore of the barrel-like rubber tube, and the fifth through hole 221 may be provided by an inner bore of the tubular member.

Referring to Figs. 6a to 6c, a cross-section of the second cavity 240 may optionally comprise a first reference axis A1 and a second reference axis A2, which both pass through the center of the cross-section, and the first reference axis A1 is perpendicular to the second reference axis A2. The cross-section may be symmetrical not only about the first reference axis A1 but also about the second reference axis A2. Moreover, a dimension of the cross-section along the first reference axis A1 may differ from a dimension of the cross-section along the second reference axis A2. Preferably, the cross-section shape of the second cavity 240 complements an outer contour of the proximal portion 12 of the implantable medical device 1. The different dimensions of the cross-section of the second cavity 240 along the first and second reference axes A1 and A2 enable the proximal portion 12 to transfer a torque after it is inserted into the second cavity 240.

Further, since the cross-section of the second cavity 240 is identical in shape on opposite sides of the first reference axis A1 and on opposite sides of the second reference axis A2, it can be easily fabricated and facilitates torque transmission, and it can be ensured that the proximal portion 12 of the implantable medical device 1 can be smoothly inserted into the second cavity 240 coaxially and centrally, with less friction or interference between the safety wire 5 and the guide chamfer 243 and a through hole such as the fifth through hole 221 during the insertion of the proximal portion 12 into the second cavity 240.

The docking of the implantable medical device 1 with the docking member 24 is not limited to being accomplished by the non-circular second cavity 240 as illustrated in the above embodiment. Referring to Figs. 9 and 10, in another exemplary example, the docking member 24 comprises, at its distal end, a plurality of notches 244, which enable docking of the proximal portion 12 of the implantable medical device 1. The notches 244 are arranged circumferentially around the axis of the docking member 24, and the implantable medical device 1 can be brought into engagement with the notches 244 along the axis of the docking member 24 and thus restrained from circumferential rotation. Preferably, the notches 244 are equidistantly arranged circumferentially around the axis of the docking member 24. It will be appreciated that the proximal portion 12 of the implantable medical device 1 may have one or more teeth complementary to at least one of the notches 244. Moreover, the number of notches 244 may be larger than or equal to that of teeth. The teeth of the proximal portion 12 may engage in respective notches 244, thereby docking the implantable medical device 1 to the docking member 24. Once the two are docked together, they are restrained from relative circumferential rotation and able to transfer a torque.

When there is at least one tooth, and the number of the teeth is less than the number of the notches 244, the tooth may engage with one notch 244 by male/female mating to enable torque transfer between the docking member 24 and the implantable medical device 1. Since the tooth may engage with any one of the notches 244, easier circumferential alignment can be achieved between the implantable medical device 1 and the docking member 24.

When the teeth and the notches 244 are multiple in number and equal in quantity, each tooth may engage with corresponding notch 244 by male/female mating to enable torque transfer between the docking member 24 and the implantable medical device 1.

In embodiments described herein, there is also provided a method of assembling a torque transmission mechanism 2. This method can be used to assemble the torque transmission mechanism 2 as discussed above and includes the steps described below.

Step S1: sleeving the torque transmission member 21 over the torque receiver 22.

Step S2: performing a torque test on the torque transmission member 21 and the torque receiver 22 by applying a torque exceeding the rated value to the torque transmission member 21. If the torque transmission member 21 rotates around the torque receiver 22, the torque test is passed.

Step S3: after passing the torque test, couple the limiting member 23 to both the torque transmission member 21 and the torque receiver 22 so that a position of the torque transmission member 21 is limited relative to the torque receiver 22 on the axis of the torque receiver 22. Optionally, the coupling of the limiting member 23 and the torque receiver 22 may be accomplished indirectly through the docking member 24. In one exemplary example, coupling the limiting member 23 to the torque receiver 22 includes the steps of: sleeving the barrel section 231 of the limiting member 23 onto the outer circumference of the support portion 241 of the docking member 24; and then fixing the limiting member 23 to the docking member 24 by welding, melting or gluing (in some preferred examples, this may be accomplished by snap engagement, threaded connection or the like). Once the docking member 24 is coaxially fixedly coupled to the torque receiver 22, a coupling is formed between the limiting member 23 and the torque receiver 22.

Optionally, the method of assembling a torque transmission mechanism 2 may further include the following steps:
Step Sa1: before the torque transmission member 21 is sleeved over the torque receiver 22, the coupling member 25 is assembled with and coupled to the proximal end of the torque transmission member 21. Preferably, the coupling member 25 is fixedly coupled to the proximal end of the torque transmission member 21, for example, by welding, melting or gluing.

Step Sa2: after the torque test is passed, the docking member 24 arranged at the distal end of the torque receiver 22 is fixedly coupled to the limiting member 23, and then the first protruding portion 72 of the limiting member 23 is engaged into the first limiting groove 71 of the coupling member 25 so that the limiting member 23 is rotatably coupled to the coupling member 25. Step Sa2 can be specifically configured according to the structures of the docking member 24 and the limiting member 23.

In step Sa2, engaging the first protruding portion 72 of the limiting member 23 into the first limiting groove 71 of the coupling member 25 so that the limiting member 23 is rotatably coupled to the coupling member 25 may specifically include the steps of: the limiting member 23 is movably sleeved over the body 250 of the coupling member 25 from the proximal end to the distal end; after the first protruding portion 72 comes into abutment with the stop shoulder 251, the limiting block 252 is mounted to the body 250 from the distal end to the proximal end until it is located in the vicinity of, or abuts against, the first protruding portion 72; and then fixing the limiting block 252 to the body 250 and the inner sheath 4, for example, by welding, melting or gluing. As a result, the first limiting groove 71 is formed between the stop shoulder 251 and the limiting block 252, with the first protruding portion 72 being engaged in the first limiting groove 71. Thus, the limiting member 23 is rotatably coupled to the coupling member 25 while being restrained from axial movement relative to the coupling member 25.

It should be noted that the method can be used during the fabrication, testing or preoperative preparation of the torque transmission mechanism 2. Thus, it is unnecessary for a surgeon to assemble the torque transmission mechanism 2 on site during a surgical procedure, effectively reducing the time required by the procedure and increasing its success.

Use of the torque transmission mechanism 2 of the present embodiment with a delivery device in specific application is further described below as an exemplary example.

First of all, the implantable medical device 1 is assembled into the second cavity 240 of the docking member 24 (refer to the above description of the embodiment made in connection with Figs. 6a to 6c). Alternatively, the proximal portion 12 of the implantable medical device 1 is engaged with the notches 244 (refer to the above description of the embodiment made in connection with Figs. 9 and 10). The inner sheath 4 is pulled proximally (i.e., to the right as viewed in the orientation of Fig. 1) so that the torque-limiting structure 2 and the implantable medical device 1 are received in the casing 6 configured for housing the implantable medical device 1.

The casing 6 is inserted into a guide sheath, with which an access has been established. When the casing 6 is entirely advanced out of the guide sheath into the right atrium, the outer sheath 3 is manipulated to further advance the casing 6 smoothly through the tricuspid valve into the right ventricle. A contrast agent is injected to allow observing the location of the casing 6 in the right ventricle, determining whether the casing 6 is oriented perpendicular to the ventricular septum and, if not, tuning it into such an orientation. The contrast agent may overflow from a number of through holes in the torque-limiting structure 2 (e.g., the first through hole 242, the third through hole 233, the fourth through hole 211, the fifth through hole 221 and the sixth through hole 253), then pass through a clearance between the torque-limiting structure 2 and the casing 6, and is finally ejected from the casing 6 through its distal end (i.e., the left hand end thereof as viewed in the orientation of Fig. 3). The inner sheath 4 is pushed to urge the distal portion 11 of the implantable medical device 1 entirely out of the casing 6, and then turned (e.g., clockwise) to cause the docking member 24 and the implantable medical device 1 to rotate. With the aid of X-ray images, an amount of rotation of the implantable medical device 1 is observed. For example, if it has been rotated by two revolutions, the inner sheath 4 may be further turned to see whether the implantable medical device 1 is further rotated. Generally, after the implantable medical device 1 is rotated by two revolutions, the distal portion 11 will be screwed to a more or less sufficient depth and subject to a strong resistive torque from the ventricular septum. At this point, the torque-limiting structure 2 will act by the torque transmission member 21 being caused to rotate around the torque receiver 22 to avoid a greater torque from being transferred to the docking member 24.

The casing 6 and the torque-limiting structure 2 are retracted into the right atrium or inferior vena cava, with only the safety wire 5 being maintained coupled to the implantable medical device 1. Electrical performance of the implantable medical device 1 is then tested, and if it passes the test, the safety wire 5 may be separated from the implantable medical device 1 (e.g., by rotating the safety wire 5 counterclockwise until it is detached from the proximal portion 12 of the implantable medical device 1). Otherwise, if the electrical performance is found to be unsatisfactory, the torque-limiting structure 2 and the casing 6 may be again advanced over the safety wire 5 into the right ventricle and the implantable medical device 1 is again proximally engaged with the docking member 24. After that, the inner sheath 4 may be rotated counterclockwise to counterclockwise unscrew the distal portion 11 of the implantable medical device 1 off the ventricular septum, followed by repositioning and implantation of the implantable medical device 1. This process may be repeated until designed electrical performance has been confirmed according to testing results. Subsequently, the safety wire 5 may be again separated from the implantable medical device 1, and the entire delivery device and guide sheath may be withdrawn, completing the procedure.

In summary, the present invention provides a torque transmission mechanism, a method of assembly thereof and a delivery device for an implantable medical device. The torque transmission mechanism includes a torque transmission member, a torque receiver and a limiting member. The torque transmission member is sleeved over an outer circumference of the torque receiver along an axis thereof and configured to transfer a torque to the torque receiver. If a torque on the torque transmission member does not exceed a rated value, a torque on the torque receiver is equal to the torque on the torque transmission member. Otherwise, if the torque on the torque transmission member exceeds the rated value, the torque transmission member rotates around the torque receiver so that the torque on the torque receiver does not exceed the rated value. The limiting member is configured to limit axial positions of the torque transmission member and the torque receiver. With this arrangement, based on the configuration of torque transmission member and the torque receiver, the torque transmission member, when a torque thereon exceeds the rated value, will rotationally slip on and around the torque receiver, thereby ensuring that the torque transmission member can only transfer a torque not exceeding the rated value to the torque receiver. Additionally, it is unnecessary to separate the torque transmission member from the torque receiver, preventing possible introduction of a gap which might occur if the separation fails. The provision of the limiting member ensures that the torque transmission member will not axially separate from the torque receiver, thus ensuring reliability of the entire torque transmission mechanism and eliminating the risk of scratching of myocardial tissue that might be caused by separation of the torque transmission member from the torque receiver.

It should be noted that the foregoing embodiments may be combined in any suitable combination. The description presented above is merely that of some preferred embodiments of the present invention and is not intended to limit the scope thereof in any sense. Any and all changes and modifications made by those of ordinary skill in the art based on the above teachings fall within the scope of the invention.

## Claims

1. A torque transmission mechanism, configured to cause an implantable medical device to rotate so as to implant the implantable medical device at a target site, wherein the torque transmission mechanism comprises a torque transmission member, a torque receiver and a limiting member,
wherein the torque transmission member is sleeved over an outer circumference of the torque receiver along an axis of the torque receiver, wherein the torque transmission member is configured to receive and transfer a torque to the torque receiver, wherein when a torque on the torque transmission member does not exceed a rated value, the torque transmission member drives the torque receiver to rotate in synchronization therewith, wherein when the torque on the torque transmission member exceeds the rated value, the torque transmission member rotates around the torque receiver so that a torque on the torque receiver does not exceed the rated value, wherein the limiting member is configured to limit a position of the torque transmission member relative to the torque receiver on the axis of the torque receiver.

2. The torque transmission mechanism according to claim 1, further comprising a docking member and a coupling member, wherein the docking member is disposed at a distal end of the torque receiver along the axis of the torque receiver, wherein the coupling member is disposed at a proximal end of the torque transmission member along an axis of the torque transmission member.

3. The torque transmission mechanism according to claim 2, wherein the limiting member is coupled to each of the docking member and the coupling member, wherein the docking member and/or the coupling member is/are rotatably coupled to the limiting member, and wherein the docking member limits a position of the limiting member on the axis of the torque receiver and the coupling member limits a position of the limiting member on the axis of the torque transmission member.

4. The torque transmission mechanism according to claim 3, wherein one of the coupling member and the limiting member comprises a circumferential first limiting groove, with the other comprising a circumferential first protruding portion, wherein the first protruding portion is engaged in the first limiting groove, thereby limiting a relative position of the coupling member and the limiting member on the axis of the torque transmission member, and/or
wherein one of the docking member and the limiting member comprises a circumferential second limiting groove, with the other comprising a circumferential second protruding portion, wherein the second protruding portion is engaged in the second limiting groove, thereby limiting a relative position of the docking member and the limiting member on the axis of the torque receiver.

5. The torque transmission mechanism according to claim 3, wherein the docking member is fixedly coupled to the limiting member by snap engagement or threadedly engagement, wherein the coupling member comprises a circumferential first limiting groove, wherein the limiting member comprises a circumferential first protruding portion that is engaged in the first limiting groove, so that the limiting member is around and rotatably coupled to the coupling member.

6. The torque transmission mechanism according to claim 2 or 3, wherein the limiting member comprises a barrel section, wherein the barrel section surrounds and shields the torque transmission member along the axis of the torque transmission member.

7. The torque transmission mechanism according to claim 6, wherein the docking member comprises a support portion extending radially thereof, wherein a distal end of the barrel section is attached to the support portion, wherein the support portion comprises a first through hole, and/or the barrel section comprises a second through hole, wherein the limiting member further comprises a tapered section at a proximal end of the barrel section, wherein the tapered section is tapered away from the support portion and comprises a third through hole, wherein the first through hole and/or the second through hole is/are brought into communication with the third through hole through a first cavity of the barrel section.

8. The torque transmission mechanism according to claim 1, further comprising a docking member disposed at a distal end of the torque receiver, wherein the docking member comprises a second cavity for docking with the implantable medical device, wherein a distal end of the second cavity is open to allow the implantable medical device to be moved into or out of the docking member along an axis thereof, wherein the second cavity configured to, when a proximal end portion of the implantable medical device is accommodated in the second cavity, limit a circumferential rotation of the implantable medical device.

9. The torque transmission mechanism according to claim 8, wherein a cross-section of the second cavity comprises a first reference axis and a second reference axis that pass through a center of the cross-section, wherein the first reference axis is perpendicular to the second reference axis, wherein the cross-section is symmetrical about each of the first reference axis and the second reference axis, wherein a dimension of the cross-section along the first reference axis is different from a dimension of the cross-section along the second reference axis.

10. The torque transmission mechanism according to claim 1, further comprising a docking member disposed at a distal end of the torque receiver, wherein the docking member comprises a plurality of notches for docking with the implantable medical device, wherein the notches are arranged circumferentially around an axis of the docking member, wherein the notches are configured to engage the implantable medical device along the axis of the docking member so as to limit a circumferential rotation of the implantable medical device.

11. The torque transmission mechanism according to claim 1, wherein an interference fit is formed between the torque transmission member and the torque receiver, wherein when the torque on the torque transmission member does not exceed the rated value, the torque transmission member and the torque receiver are stationary relative to each other, wherein when the torque on the torque transmission member exceeds the rated value, the torque transmission member overcomes friction between the torque transmission member and the torque receiver and hence rotates around the torque receiver.

12. The torque transmission mechanism according to claim 11, wherein the torque transmission member comprises a torque spring, wherein the torque receiver comprises a cylindrical outer circumferential wall, and wherein the torque spring is wound on the outer circumferential wall.

13. The torque transmission mechanism according to claim 1, wherein the torque transmission member comprises a fourth through hole axially extending therethrough, wherein the torque receiver comprises a fifth through hole axially extending therethrough, wherein the fourth and fifth through holes are configured for passage of a safety wire therethrough.

14. A method of assembling a torque transmission mechanism as defined in any one of claims 1 to 13, comprising:
disposing the torque transmission member over the torque receiver;
performing a torque test on the torque transmission member and the torque receiver by applying a torque exceeding the rated value to the torque transmission member, wherein if the torque transmission member rotates around the torque receiver, the torque test is passed; and
after passing the torque test, coupling the limiting member to each of the torque transmission member and the torque receiver so that a position of the torque transmission member is limited relative to the torque receiver on the axis of the torque receiver.

15. The method of assembling a torque transmission mechanism according to claim 14, further comprising:
wherein before the torque transmission member is sleeved over the torque receiver, the coupling member is assembled with and coupled to a proximal end of the torque transmission member; and
wherein after the torque test is passed, the docking member at a distal end of the torque receiver is fixedly coupled to the limiting member and the first protruding portion of the limiting member is engaged into the first limiting groove of the coupling member so that the limiting member is rotatably coupled to the coupling member.

16. A delivery device for an implantable medical device, comprising a torque transmission mechanism as defined in any one of claims 1 to 13, an outer sheath, an inner sheath and a safety wire,
wherein the inner sheath is movably disposed inside the outer sheath, wherein the safety wire is movably disposed inside each of the inner sheath and the torque transmission mechanism, wherein the inner sheath is coupled to the torque transmission member of the torque transmission mechanism and is configured to actuate the torque receiver of the torque transmission mechanism by transferring a torque to the torque transmission member,
wherein a distal end of the torque transmission mechanism is configured to be detachably coupled to the implantable medical device, and wherein the torque receiver of the torque transmission mechanism is coupled to the implantable medical device so as to limit a circumferential rotation of the implantable medical device,
wherein the safety wire is configured to be detachably coupled to the implantable medical device.

17. An implantable medical system, comprising the delivery device of claim 16 and an implantable medical device, wherein a distal portion of the implantable medical device is configured for implantation at a target site, wherein a proximal portion of the implantable medical device is configured to be detachably coupled to the delivery device, thereby allowing the implantable medical device to be delivered to and implanted at the target site by the delivery device.
